# EUROPÄISCHE PATENTANMELDUNG

(11) **EP 4 595 949 A1**
(43) Veröffentlichungstag der Anmeldung: **06.08.2025**
(21) Anmeldenummer: 25155245.1
(22) Anmeldetag: 31.01.2025
(51) Int. Cl.: A61K 9/00, A23L 2/00, A61K 9/20, A61K 31/132, A61K 31/352, A61K 31/353, A61K 31/385, A61K 31/405, A61K 31/4375, A61K 31/5685, A61K 31/685, A61K 35/00, A61K 36/00

(54) **SCHMELZTABLETTE, INSBESONDERE ZUR VERWENDUNG ALS NAHRUNGSERGÄNZUNGSMITTEL**

(30) Priorität: 01.02.2024 DE 102024102935
(71) Anmelder: Goethe Biotechnology GmbH, 60314 Frankfurt am Main (DE); BRIU GmbH, 61462 Königstein (DE)
(72) Erfinder: ULLRICH, Michael, 60439 Frankfurt (DE); HARTENSTEIN, Günther, 76829 Landau (DE)
(74) Vertreter: Tergau & Walkenhorst Intellectual Property GmbH

(57) **Zusammenfassung**

Die Erfindung betrifft eine Schmelztablette, insbesondere zur Verwendung als Nahrungsergänzungsmittel, umfassend als Hauptbestandteile:
- einen Wirkstoff, umfassend einen oder mehrere von Berberin, Spermidin, Dehydroepiandrosteron, 5-Hydroxytryptophan, Gelée royale, Propolis, Polyen Phosphatidylcholin, Quercetin, Dihydroquercetin, Epigallocatechingallat, α-Liponsäure und/oder Myrrhe,
- ein Disaccharid
- ein Phosphatidylcholin

## Beschreibung

Die Erfindung betrifft eine Schmelztablette, insbesondere zur Verwendung als Nahrungsergänzungsmittel.

Aus der Naturmedizin oder Naturheilkunde sind eine Vielzahl von Wirkstoffen zu verschiedenartigen Behandlungszwecken bekannt. Beispielsweise findet der Wirkstoff Berberin, auch bekannt als 5,6-Dihydro-9,10-dimethoxybenzo[g]-1,3-benzodioxolo[5,6-a]chinolizinium-Chlorid, im Rahmen der alternativen Medizin bereits seit geraumer Zeit Verwendung zu verschiedenartigen Behandlungszwecken. Insbesondere wird diesem Wirkstoff eine Steigerung der Insulinsekretion zugeschrieben, und er soll die Insulinresistenz und den Fettstoffwechsel günstig beeinflussen.

Berberin ist ein Alkaloid aus der Gruppe der Isochinolinalkaloide. Es kommt unter anderem in den Pflanzen Berberitze, die dem Alkaloid den Namen gab, der Orangenwurzel und Coptis chinensis, im Wesentlichen in den Wurzeln, im Rhizom, im Stamm und in der Rinde, vor. Es hat die Struktur:

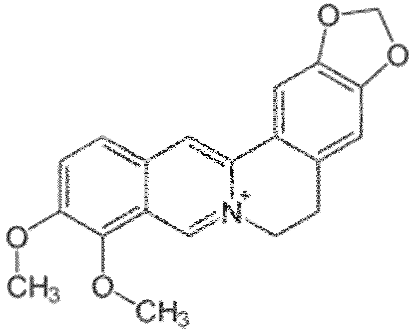

Des weiteren ist Spermidin, auch Monoaminopropylputrescin genannt, ein biogenes Polyamin und ein Zwischenprodukt bei der Bildung von Spermin aus Putrescin und decarboxyliertem S-Adenosylmethionin. Ihm wird zugeschrieben, dass Spermidin nicht nur die Zellen verjüngt, sondern auch Entzündungen dämpft und das Cholesterin im Körper senkt.

Spermidin weist folgende Strukturformeln auf:

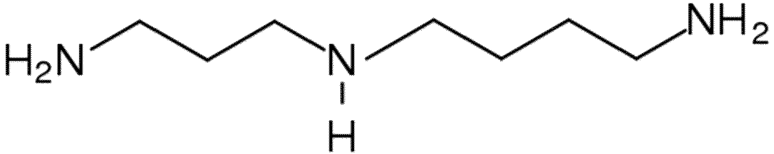

Dehydroepiandrosteron, auch als Prasteron bezeichnet, kurz DHEA, ist das am häufigsten vorkommende Steroidhormon im menschlichen Körper. In Abhängigkeit vom jeweiligen hormonellen Niveau kann es sich wie ein Estrogen oder wie ein Androgen verhalten. DHEA ist die Vorstufe sowohl für die männlichen Sexualhormone als auch für weiblichen Sexualhormone. Es hat die chemische Formel C₁₉H₂₈O₂ und die Strukturformel

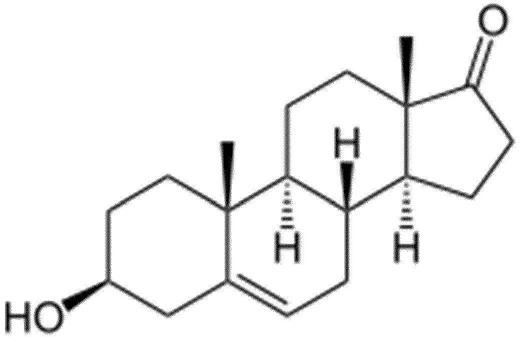

5-Hydroxytryptophan, genauer L-5-Hydroxytryptophan [Synonym:-5-Hydroxytryptophan], Freiname: Oxitriptan, ist eine nicht-proteinogene α-Aminosäure mit lipophiler aromatischer Seitenkette. Es ist ein Zwischenprodukt bei der Serotoninsynthese aus L-Tryptophan in Organismen. Seine Wirkung wird oft als stimmungsaufhellend, beruhigend und gewichtsreduzierend beschrieben. Es hat die chemische Formel C₁₁H₁₂N₂O₃ und die Strukturformel

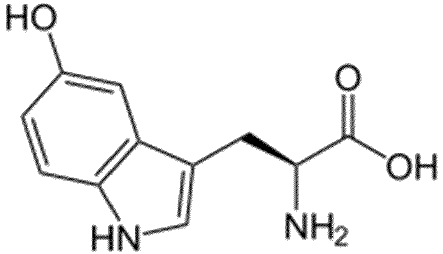

Gelée royale, Weiselfuttersaft oder Bienenköniginnenfuttersaft ist der Futtersaft, mit dem die Honigbienen ihre Königinnen aufziehen. Der Verzehr von Gelée Royale soll die Lebenserwartung, Libido und Fruchtbarkeit erhöhen, das Immunsystem stärken, die Vitalität steigern und bei Wechseljahrsbeschwerden helfen. Seine wichtigsten Inhaltsstoffe sind:
10-23 % Zucker
60-70 % Wasser
9-18 % Proteine und Aminosäuren
4-8 % Fette
Thiamin, Riboflavin, Pyridoxin, Niacin, Pantothensäure, Biotin, Folsäure, Sterine, Biopterin, Juvenilhormon und Neopterin, Mineralstoffe und Spurenelemente.
4-Hydroxybenzoesäuremethylester als natürliches Konservierungsmittel

Propolis (auch Kittharz, Bienenharz, Bienenleim genannt) ist die Substanz, die Bienen von den Knospen und der Rinde der Laubbäume sammeln und mit Wachs, Pollenanteilen und Speichelsekreten anreichern. Schädliche freie Radikale können durch Phenol und Flavonoide in Propolis gebunden werden. Das hilft dem Stoffwechsel und steigert das Wohlbefinden. Durch ihre antioxidative Wirkung wirkt sich Propolis auf das Immunsystem aus.

Phosphatidylcholine (PC), insbesondere als polyene Phosphatidylcholine, in Sumem auch als Lecithine bezeichnet, sind Phospholipide, die Ester aus Fettsäuren, Glycerin, Phosphorsäure und Cholin darstellen. In der praktischen Verwendung im Bereich der Lebensmittel und der Pharmazie versteht man unter Lecithin hingegen technisch gewonnene Produkte, die neben Phosphatidylcholinen als Hauptbestandteilen auch andere Phospholipide und Begleitstoffe enthalten. Phosphatidylcholine sind Bestandteile der Zellmembran tierischer und pflanzlicher Lebewesen. In den Membranen der meisten Bakterien kommen sie hingegen nicht vor. Sie sind Begleitstoffe in Fetten und Ölen und besonders reich in Eidotter und Zellen pflanzlicher Samen vorhanden.

Quercetin ist ein gelber Naturstoff aus der Gruppe der Polyphenole und Flavonoide und das Oxidationsprodukt des Anthocyanidins Cyanin. Der sekundäre Pflanzenstoff Quercetin beeinflusst den Histamin-Stoffwechsel und ist zudem ein wertvolles Antioxidans. Es wurde vor allem in letzter Zeit bekannt für seine Fähigkeit, saisonale Allergiesymptome zu lindern, da es antihistaminische und antivirale Eigenschaften besitzt und den oxidativen Stress in den Arterien reduziert. Es hat die chemische Formel C₁₅H₁₀O₇ und die Strukturformel

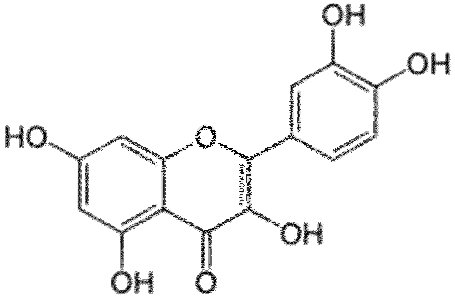

Dihydroquercetin, auch als Taxifolin bekannt, ist eine natürlich vorkommende organische Verbindung, die chemisch zu den Flavanonolen innerhalb der Stoffgruppe der Flavonoide zählt. Antioxidative Wirkungen auf C₁₅H₁₂O₇ Stoffwechselfunktionen beim Menschen sind, wie für viele Flavonoide, nachgewiesen. Das Vorhandensein einer Doppelbindung in Nachbarschaft zu einer Hydroxygruppe (Enol-Struktur) und somit eine Möglichkeit zur Stabilisierung energetisch ungünstiger Zustände scheint also ein wesentliches Merkmal antioxidativer Flavonoide zu sein. Taxofolin hat die chemische Formel C₁₅H₁₂O₇ und die Strukturformel 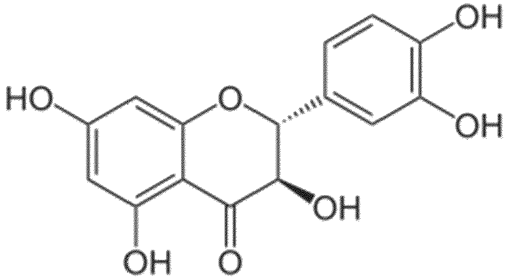 EGCG, Epigallocatechingallat, ordnet sich den Catechinen zu, welche der Gruppe der Flavoide, sekundären Pflanzenstoffen zuzurechnen sind. Catechine zeichnen sich durch ein hohes antioxidatives Potential aus, was bedeutet, dass sie Oxidationen anderer Stoffe verlangsamt oder komplett verhindert. Es ist ein Carbonsäureester der Gallussäure mit dem Alkohol und Catechin Epigallocatechin. Das Antioxidans stellt die Mehrheit aller Catechine des grünen Tees dar. Im schwarzen Tee ist der Anteil an Catechinen deutlich geringer, da aufgrund der Fermentation die Catechine zu oligomeren Theaflavinen reagieren. Es hat die chemische Formel C₂₂H₁₈O₁₁ und die Strukturformel

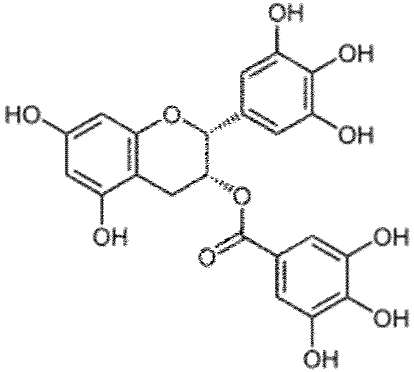

α-Liponsäure (abgekürzt LA vom englischen lipoic acid oder ALA vom englischen alpha lipoic acid; anderer Name Thioctsäure) ist eine schwefelhaltige Fettsäure. In ihrer natürlichen (R)-Form kommt sie als Coenzym in den Mitochondrien fast aller Eukaryoten vor und spielt eine wichtige Rolle im Energiestoffwechsel. Sie wird als Arzneistoff zur Behandlung von Lebererkrankungen und bei peripheren Polyneuropathien eingesetzt. Sie hat die chemische Formel C₈H₁₄O₂S₂ und die Strukturformel

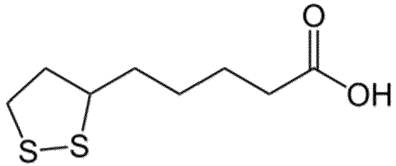

Myrrhe oder auch Myrrhenharz ist das aromatische Gummiharz von mehreren Arten der Gattung Commiphora ("Myrrhenstrauch") aus der Familie der Balsambaumgewächse, in erster Linie Commiphora myrrha (Synonyme: Commiphora molmol, früher auch Balsamea myrrha Engl.). Es wird bereits seit 2000 Jahren vor allem äusserlich als Salböl gegen Husten und Bauchschmerzen angewendet.

Aufgrund ihrer anerkannt guten Wirkungsweisen besteht für diese Wirkstoffe das Bedürfnis, sie für den einfachen und alltäglichen Gebrauch noch besser als bisher verfügbar und einfach nutzbar zu machen. Insbesondere sollte dabei eine Verfügbarkeit in vergleichsweise großen Mengen ermöglicht werden, was angesichts der Knappheit und des hohen Preises dieser Produkte nur bedingt möglich erscheint.

Der Erfindung liegt daher die Aufgabe zugrunde, eine für diese Zwecke besonders geeignete Darreichungsform anzugeben.

Diese Aufgabe wird erfindungsgemäß gelöst durch eine Schmelztablette, umfassend als Hauptbestandteile:
- einen Wirkstoff
- ein Disaccharid
- ein Phosphatidylcholin

Eine solche Schmelztablette ist insbesondere und besonders bevorzugt zur Verwendung als Nahrungsergänzungsmittel vorgesehen.

Die Erfindung geht dabei von der Überlegung aus, dass im Hinblick auf die oben genannten Auslegungsziele eine Darreichung des jeweiligen Wirkstoffs auf besonders effiiziente Weise erfolgen sollte, so dass der Wirkstoff dem menschlichen Organismus besonders zielgenau zugeführt werden kann. Damit ist ein besonders sparsamer Umgang mit dieser knappen Ressource bei hoher Ausnutzung des therapeutischen Potentials möglich. Um dies zu ermöglichen, ist erfindungsgemäß vorgesehen, den Wirkstoff in der Form einer Schmelztablette bereitzustellen. Eine solche Schmelztablette soll sich nach Aufnahme direkt im Mundbereich auflösen, wobei der Wirkstoff freigesetzt wird und anschließend direkt über die Mundschleimhaut (Mucosa) aufgenommen werden kann. Damit erfolgt die Zuführung in das Körpersystem auf hoch effiziente Weise und unter Umgehung der Leber.

Damit kann auch der so genannte "First-Pass-Effekt" umgangen werden. Dieser beschreibt die Umwandlung eines Wirkstoffes während dessen erster Passage ("first pass") durch die Leber. Durch die dabei stattfindende Umwandlung (Metabolisierung) kann ein wirksamer oder unwirksamer Metabolit entstehen.

Als Wirkstoff ist dabei gemäß einem Aspekt der Erfindung einer oder mehrere von Berberin, Spermidin, Dehydroepiandrosteron, 5-Hydroxytryptophan, Gelée royale, Propolis, Polyen Phosphatidylcholin, Quercetin, Dihydroquercetin, Epigallocatechingallat, α-Liponsäure und/oder Myrrhe vorgesehen. Die Verwendung eines jeden dieser Wirkstoffe in der vorliegenden Schmelztablette wird jeweils als eigenständig erfinderisch angesehen.

Um eine solche, erfindungsgemäß vorgesehene Schmelztablette zur Verabreichung eines oder mehrerer der genannten Wirkstoffe bereitstellen zu können, muss dieser Wirkstoff geeignet in Zusatz- oder Trägerstoffe eingebunden werden, die einerseits eine formstabile Tablettenform mit einer gewissen Haltbarkeit und Lagerfähigkeit ermöglichen, die andererseits aber auch im Mundbereich aufgelöst werden können, so dass der Wirkstoff freigesetzt wird und über die Mucosa aufgenommen werden kann. Um dies zu ermöglichen, ist vorgesehen, zusätzlich zum eigentlichen Wirkstoff ein Disaccharid und ein Phosphatidylcholin, üblicherweise auch bezeichnet als Lecithin, als weitere Bestandteile für die Schmelztablette zu nutzen.

In vorteilhafter Ausgestaltung ist dabei als Disaccharid Trehalose vorgesehen. Trehalose ist ein Disaccharid, das aus zwei α,α'-1,1-glycosidisch verknüpften Glucose-Molekülen besteht. Daher ist sein systematischer Name 1-α-Glucopyranosyl-1-α-Glucopyranosid, in der Kurzformel: Glc α(1→1)α Glc. Da beide anomeren C-Atome an der O-glycosidischen Bindung beteiligt sind, gehört Trehalose zu den nicht-reduzierenden Zuckern.

In alternativer oder zusätzlicher vorteilhafter Weiterbildung ist als Phosphatidylcholin Sonnenblumenlecithin vorgesehen. Gerade die Kombination dieser Zusatzstoffel, also der Trehalose und des Lecithins, ist ganz besonders günstig für die Verstoffwechselung in der Mundschleimhaut und wird als eigenständig erfinderisch angesehen.

Um die genannten Wirkungsweisen besonders zuverlässig bereitzustellen, ist der Gewichtsanteil des Phosphatidylcholin vorteilhafterweise um mindestens 50 % höher als der Gewichtsanteil des Disaccharids, und beträgt vorzugsweise mindestens das Doppelte.

Bei der Herstellung der als Grundstoff für die Schmelztablette vorgesehenen Zusammensetzung ist gemäß einem als eigenständig erfinderisch angesehenen Aspekt die Ultraschall-Extraktion vorgesehen, um die jeweiligen Wirkstoffe geeignet zu extrahieren. Nach der Extraktion sollte nicht filtriert werden, da damit eine Abschwächung der Wirkkräfte einhergehen könnte. Stattdessen ist gemäß einem Aspekt der Erfindung vorgesehen, das Extrakt ausschließlich durch Sedimentation (Zentrifugation) von enthaltenen Feststoffen zu befreien.

Während der Extraktion wird gemäß einem Aspekt der Erfindung, beispielsweise mittels eines Rührwerks, für eine gute Verteilung und Homogenisierung des Wirkstoffextrakts gesorgt, um ihn besser aufschliessen zu können.

Gemäß einem als eigenständig erfinderisch angesehenen Aspekt erfolgt die Herstellung der Schmelztablette unter Rückgriff auf das Konzept der Lipoliophilisation. Dabei werden Fette gefriergetrocknet, was unter normalen Umständen eigentlich nicht funktioniert. Dazu wird in einem ersten Schritt, durch sorgfältiges Rühren, eine möglichst komplett homogene Flüssigkeit oder Emulsion mit sämtlichen der einzelnen Inhaltsstoffe hergestellt. Dies kann bei dem Prozess der Lipoliophilisation als der wichtigste Schritt angesehen werden. Je feiner nämlich die entstandene homogene Emulsion, um so leichter lässt sich der Prozeß der Lipoliophilisation abbilden.

Der Trägerstoff Trehalose wird anschließend unter anderem hierfür verwendet, ein Gerüst für die Fettmoleküle zu bauen. Hierdurch wird es ermöglicht das Wasser bei dem Liophilsationsprozesses zu sublimieren und die Fettmolekühle an die Trehalose zu binden. Die entstandene Liposom-Trehalose Mischung kann wie beschriebenbei der Verabreichung über die Mundschleimhaut (Mukosa) extrem gut aufgenommen werden.

Die Wirkung der Schmelztablette kann in etwa wie folgt charakterisiert werden:
Durch die genannten Zusatzstoffe findet eine lipophile Aufbereitung der Ausgangssubstanzen zur Schmelztablette statt, und durch diese wird der Wirkstoff direkt über die Mundschleimhaut aufgenommen. Das Disaccharid, also vorzugsweise die Trehalose, liefert dabei eine Art Stützstruktur, die die Raumform und Stabilität der Schmelztablette ermöglicht. Bei der Aufnahme in den Mundraum zersetzt der Speichel aber enzymatisch das Disaccharid, so dass der Wirkstoff zur Resorption über die Mundschleimhaut freigesetzt wird.

Das (Sonnenblumen-) Lecithin wird dabei ebenfalls über den Speichel (Lipasen) gelöst und über die Mundschleimhaut resorbiert.

Die Einnahme der Schmelztablette sollte gesondert von den Mahlzeiten genommen werden, und mindestens 15 Minuten im Mundraum ohne weitere Nahrungsaufnahme verweilen. In diesem Zeitraum kann die komplette Resorption im Mund stattfinden.

## Patentansprüche

1. Schmelztablette, insbesondere zur Verwendung als Nahrungsergänzungsmittel, umfassend als Hauptbestandteile:
- einen Wirkstoff, umfassend einen oder mehrere von Berberin, Spermidin, Dehydroepiandrosteron, 5-Hydroxytryptophan, Gelée royale, Propolis, Polyen Phosphatidylcholin, Quercetin, Dihydroquercetin, Epigallocatechingallat, α-Liponsäure und/oder Myrrhe,
- ein Disaccharid
- ein Phosphatidylcholin

2. Schmelztablette nach Anspruch 1, bei der als Disaccharid Trehalose vorgesehen ist.

3. Schmelztablette nach Anspruch 1 oder 2, bei der als Phosphatidylcholin Sonnenblumenlecithin vorgesehen ist.

4. Schmelztablette nach einem der Ansprüche 1 bis 3, bei der der Gewichtsanteil des Phosphatidylcholin um mindestens 50 % höher ist als der Gewichtsanteil des Disaccharids, vorzugsweise mindestens das Doppelte beträgt.
